# NEUE EUROPÄISCHE PATENTSCHRIFT

(11) **EP 0 279 303 B2**
(45) Veröffentlichungstag und Bekanntmachung des Hinweises auf die Entscheidung über den Einspruch: **08.02.1995**
(45) Hinweis auf die Patenterteilung: 06.05.1992
(21) Anmeldenummer: 88101756.0
(22) Anmeldetag: 06.02.1988
(51) Int. Cl.: C07C 69/54, C07C 67/08, C07C 67/26, C08F 20/28

(54) **Strahlungshärtbare Acrylate**
Radiation-curable acrylates
Acrylates durcissables par rayonnement

(30) Priorität: 11.02.1987 DE 3704098
(43) Veröffentlichungstag der Anmeldung: 24.08.1988
(73) Patentinhaber: BASF Aktiengesellschaft, 67063 Ludwigshafen (DE)
(72) Erfinder: Beck, Erich, Dr., D-6800 Mannheim 31 (DE); Weiss, Wolfram, Dr., D-6704 Mutterstadt (DE); Schmidt, Horst, Dr., D-6800 Mannheim 1 (DE)

(56) Entgegenhaltungen:
- EP-A- 0 126 341
- EP-A- 0 222 059
- DE-A- 2 838 691
- DE-A- 3 241 264
- DE-A- 3 316 592
- DE-A- 3 316 593
- DE-B- 2 215 493
- FR-A- 2 237 875

## Beschreibung

Die vorliegende Erfindung betrifft strahlungshärtbare Acrylate, erhältlich durch gleichzeitige Umsetzung von
A) 1 Äquivalent eines 2-bis 6-wertigen oxalkylierten C₂- bis C₁₀-Alkohols mit
B) 0,05 bis 1 Äquivalent einer 2- bis 4-wertigen C₃- bis C₃₆-Carbonsäure oder deren Anhydride und
C) 0,1 bis 1,5 Äquivalenten Acrylsäure und/oder Methacrylsäure
sowie Umsetzung der überschüssigen Carboxylgruppen mit der äquivalenten Menge einer Epoxidverbindung, ein Verfahren zu deren Herstellung und deren Verwendung in strahlungshärtbaren Überzugsmassen.

Strahlungshärtbare Bindemittel auf Basis von acrylgruppenhaltigen Polyestern sind bekannt. Insbesondere in Hinsicht ihrer lösungsmittelfreien und schnellen Verarbeitbarkeit sind derartige Lackharze von großem Interesse.

Die Lösungsmittelfreiheit dieser Systeme ersparen den Aufwand zum Ablüften und Aufarbeiten der Lösungsmittel. Zudem wird die Emissionsgefahr durch Lösungsmittel wesentlich vermindert.

Für eine wirtschaftliche Verarbeitbarkeit sind seitens der Bindemittel im allgemeinen neben niedrigen Rohstoffkosten und einer hohen Reaktivität insbesondere auch ein geringer Reaktivverdünnerbedarf zur Einstellung geeigneter Verarbeitungsviskositäten von Bedeutung.

In der DE 32 41 264 wird eine Möglichkeit beschrieben, durch Verwendung von wäßrigen, strahlungshärtbaren Bindemitteldispersionen auf den Zusatz von Reaktivverdünnern zu verzichten. Hierzu wird ein strahlungshärtbares Acrylat einer bestimmten Zusammensetzung beschrieben, das oberflächenaktive Eigenschaften hat und dadurch die wäßrigen Dispersionen oder Emulsionen stabilisiert.

Bei der Verwendung dieser Dispersionen als Beschichtungsmittel sind aber Ablüftzeiten für das Wasser zu berücksichtigen.

Niedermolekulare Acrylester der Polyolkomponenten eines üblichen Polyesteracrylatharzes, die sich während einer sauer katalysierten Veresterungsreaktion durch gleichzeitig als Nebenreaktionen ablaufende Umesterungsreaktionen bilden, begünstigen ebenfalls einen niedrigen Verdünnerbedarf.

In der DE-OS 33 16 592 und der DE-OS 33 16 593 werden Verfahren zur Herstellung von strahlungshärtbaren Acrylaten beschrieben, wobei OH-Gruppen enthaltende Polyester mit überschüssiger Acrylsäure verestert werden und anschließend die restliche Acrylsäure durch eine Additionsreaktion mit Di- oder Polyglycidylethern zu nichtflüchtigen 2-Hydroxyacrylestern umgesetzt wird. Niedermolekulare, verdünnend wirkende Acrylester, die durch Umesterung aus Bestandteilen des Polyesters entstehen können, verbleiben im Endprodukt. Ein großer Nachteil derartiger Verbindungen ist jedoch deren erhöhte Toxizität und Flüchtigkeit, wie sie insbesondere von niedermolekularen Acrylestern bekannt sind.

Aufgabe der vorliegenden Erfindung war es deshalb, neue strahlungshärtbare Acrylate für strahlungshärtbare Überzugsmassen bereitzustellen, die deutlich verminderte Gehalte an flüchtigen und physiologisch bedenklichen Acrylverbindungen haben, deren Bedarf an Reaktivverdünnern möglichst gering ist und die sich zu hochwertigen Überzügen verarbeiten lassen.

Diese Aufgabe konnte gelöst werden durch strahlungshärtbare Acrylate, erhältlich durch gleichzeitige Umsetzung von
A) 1 Äquivalent eines 2-bis 6-wertigen oxalkylierten C₂- bis C₁₀-Alkohols mit
B) 0,05 bis 1 Äquivalent einer 2- bis 4-wertigen C₃- bis C₃₆-Carbonsäure oder deren Anhydride und
C) 0,1 bis 1,5 Äquivalenten Acrylsäure und/oder Methacrylsäure
sowie Umsetzung der überschüssigen Carboxylgruppen mit der äquivalenten Menge einer Epoxidverbindung.

Als geeignete Komponenten (A) können oxethylierte, oxpropylierte sowie gemischt oxethylierte und oxpropylierte 2- bis 6-wertige Alkohole verwendet werden, wie die Diole Ethylenglykol, Propylenglykol, Butandiol-1,4, Pentandiol-1,5, Neopentylglykol, Hexandiol-1,6, 2-Methylpentandiol-1,5, 2-Ethylbutanciol-1,4, Dimethylolcyclohexan, 1,1'-lsopropyliden-bis-(p-phenylen-oxy)-di-3-ethanol, Triole, wie Glycerin, Trimethylolethan, Trimethylolpropan, Trimethylolbutan, Tetraole, wie Pentaerythrit, Ditrimethylolpropan, Hexole, wie Erythrit und Sorbit. Bevorzugt sind 3- bis 6-wertige oxalkylierte C₃- bis C₆-Alkohole wie oxethyliertes und/oder oxpropyliertes Trimethylolpropan, Glycerin, Pentaerythrit und Sorbit.

Der Oxalkylierungsgrad liegt in der Regel zwischen 1 und 30, bevorzugt zwischen 2 und 10.

Als Komponente (B) können 2- bis 4-wertige C₃- bis C₃₆- Carbonsäuren oder deren Anhydride eingesetzt werden, wie Bernsteinsäure, Bernsteinsäureanhydrid, Glutarsäure, Glutarsäureanhydrid Adipinsäure, Sebacinsäure, Phthalsäure, Phthalsäureanhydrid, Terephthalsäure, Maleinsäure, Maleinsäureanhydrid, Fumarsäure, Citraconsäure, Tetrahydrophthalsäure, Tetrahydrophthalsäureanhydrid, Hexahydrophthalsäure, Hexachlor-endomethylentetrahydrophthalsäure, dimere Linolsäure, Trimellithsäure, Trimellithsäureanhydrid, Pyromellithsäure und Pyromellithsäureanhydrid. Bevorzugt sind Adipinsäure, Phthalsäure, Phthalsäureanhydrid, Maleinsäureanhydrid und Fumarsäure.

Geeignete Mono-, Di- oder Polyepoxidverbindungen, vorzugsweise Di- und Triepoxidverbindungen, sind epoxidierte Olefine, Glycidester von gesättigten oder ungesättigten Carbonsäuren oder Glycidether aliphatischer oder aromatischer Polyole. Bevorzugt werden die Glycidether von Butandiol, Bisphenol A und Pentaerithrit.

Die Veresterung der Komponenten (A), (B) und (C) erfolgt nach allgemein bekannten Methoden in Gegenwart von sauren Veresterungskatalysatoren, wie Schwefelsäure oder para-Toluolsulfonsäure, die in Mengen von 0,1 bis 3 Gew.%, bezogen auf die Komponenten (A), (B) und (C) eingesetzt werden, bei Temperaturen von 60 bis 140°C, wobei das entstehende Wasser azeotrop entfernt wird. Als Schleppmittel kommen aliphatische und aromatische Kohlenwasserstoffe in Frage, z.B. Alkane und Cycloalkane, wie n-Hexan, n-Heptan und Cyclohexan, Aromaten, wie Benzol, Toluol und Xylolisomere und sogenannte Spezialbenzine, welche Siedepunkte zwischen 70 und 140°C aufweisen. Besonders bevorzugte Schleppmittel sind Cyclohexan und Toluol. Die Menge des zugesetzten Kohlenwasserstoffs ist unkritisch, je nach verwendeter Apparatur kann die zugesetzte Menge zwischen der 0,1- und 2-fachen Menge des Reaktionsgemisches aus den Komponenten (A), (B) und (C) variieren. Besonders vorteilhaft ist ein Verhältnis Reaktionsgemisch zu Kohlenwasserstoff von 1:0,2 bis 1:0,8. Das eingesetzte Lösungsmittel wird nach der Veresterung, gegebenenfalls unter vermindertem Druck, aus dem Reaktionsgemisch entfernt.

Die Veresterung wird bis zu einem Umsatz von mindestens 85%, vorzugsweise 90 bis 95%, durchgeführt.

Zur Vermeidung einer vorzeitigen Polymerisation wird die Veresterung zweckmäßigerweise in Gegenwart geringer Mengen von Inhibitoren durchgeführt. Dabei handelt es sich um die üblichen, zur Verhinderung einer thermischen Polymerisation verwendeten Verbindungen, z.B. vom Typ des Hydrochinons, der Hydrochinonmonoalkylether, des 2,6-Di-tert.-butylphenols, der N-Nitrosamine, der Phenothiazine oder der Phosphorigsäureester. Sie werden im allgemeinen in Mengen von 0,001 bis 2,0 Gew.%, vorzugsweise in Mengen von 0,005 bis 0,5 Gew.%, bezogen auf die Summen der Komponenten (A), (B) und (C) eingesetzt.

Die Äquivalentverhältnisse der Komponenten (A):(B):(C) betragen 1: 0,05 bis 1: 0,1 bis 1,5 bevorzugt 1:0,1 bis 0,6:0,5 bis 0,9. Auf 1 Äquivalent des theoretischen Umsetzungsproduktes aus (A) und (B) werden 1 bis 1,5, bevorzugt 1,1 bis 1,25 Äquivalente Acrylsäure und/oder Methacrylsäure eingesetzt.

Nach der Veresterung wird im allgemeinen der Veresterungskatalysator in geeigneter Weise neutralisiert, z.B. durch Zusatz von tertiären Aminen oder Alkalihydroxiden. Die Carbonsäuregruppen des Acrylatharzes sowie die überschüssige Acrylsäure bzw. Methacrylsäure werden mit deren Säurezahl äquivalenten Menge einer der oben genannten Epoxidverbindungen bei 90 bis 130°C, vorzugsweise 100 bis 110°C, bis zu einer Säurezahl unter 5mg KOH/g umgesetzt. Dabei können zur Katalysierung der Reaktion zwischen Carboxyl- und Epoxidgruppen geeignete Verbindungen, wie tertiäre Amine, quartäre Ammoniumverbindungen oder Lewisbasen z.B. vom Typ des Thiodiglykols mitverwendet werden. Die erfindungsgemäßen Acrylate weisen z.B. Viskositäten von 0,5 bis 20 Pas, bevorzugt 1 bis 15 Pas bei 23°C auf.

Die erfindungsgemäß hergestellten Acrylate werden zur Verarbeitung im allgemeinen mit weiteren, aus der Strahlungshärtung bekannten Reaktivverdünnern versetzt. Beispielhaft seien hier lediglich genannt 4-tert.-Butylcyclohexylacrylat, Phenoxyethylacrylat, Hexandioldiacrylat, Tripropylenglykoldiacrylat, Trimethylolpropandiacrylat sowie Acrylate von alkoxylierten Diolen und Triolen. Die mittels des erfindungsgemäßen Verfahrens hergestellten Beschichtungs- und Überzugsmittel werden zweckmäßigerweise entweder durch Elektronenstrahlen oder nach Zusatz von Photoinitiatoren durch UV-Strahlen vernetzt und ergeben Filme, die den Anforderungen der Praxis voll gerecht werden.

### Beispiel 1

532,6g ethoxyliertes Trimethylolpropan mit einer OH-Zahl von 630mg KOH/g, 98g Maleinsäureanhydrid, 316,8g Acrylsäure, 437,7g Cyclohexan, 4,73g Schwefelsäure, 2,93g Hydrochinonmonomethylether, 0,95g 2,6-Di-tert.-butylkresol, 0,95g 50 gew.%ige hypophosphorige Säure und 0,028g Phenothiazin wurden zusammengegeben und bis zum Sieden erhitzt. Nachdem 93g Wasser in ca. 11 Stunden abdestilliert wurden, wurde auch das Lösungsmittel destillativ entfernt. Das Reaktionsgemisch wies eine Säurezahl von 38,8mg KOH/g auf. Anschließend wurden 105,7g Diglycidylether von Bisphenol A (Epoxid-Äquivalentgewicht 186g/mol) und 17,9g Tributylamin zugegeben. Bei 105 bis 110°C wurde die Reaktion weitergeführt, bis nach ca. 8 Stunden eine Säurezahl von 4,4mg KOH/g erreicht war. Die Viskosität bei 23°C betrug 4,3 Pa.s.

### Beispiel 2

Unter Anwendung der in Beispiel 1 beschriebenen zweistufigen Arbeitsweise wurden folgende Verbindungen umgesetzt:

### 1. Stufe:

532,6 g ethoxyliertes Trimethylolpropan (OH-Zahl 630 mg/g)
116,1 g Fumarsäure
316,8 g Acrylsäure
482,7 g Cyclohexan
4,82 g Schwefelsäure
2,99 g Hydrochinonmonomethylether
0,96 g 2,6-Di-tert.-butylkresol
0,96 g hypophosphorige Säure (50 gew.%ig)
0,96 g Triphenylphosphit
0,029 g Phenothiazin

| | |
|---|---|
| abdestillierte Wassermenge: | 110ml |
| Säurezahl: | 51,0mg KOH/g |

### 2. Stufe

120,5 g Diglycidylether von Bisphenol A (Epoxid-Äquivalentgewicht 186g/mol)
18,2 g Tributylamin

| | |
|---|---|
| Produkt-Viskosität (23°C ): | 12,8 Pa.s |

### Beispiel 3:

Unter Anwendung der in Beispiel 1 beschriebenen zweistufigen Arbeitsweise wurden folgende Verbindungen umgesetzt:

### 1. Stufe

532,6 g ethoxyliertes Trimethylolpropan (OH-Zahl 630mg/g)
146,1 g Adipinsäure
316,8g Acrylsäure
497,8 g Cyclohexan
4,98 g Schwefelsäure
3,10 g Hydrochinonmonomethylether
0,99 g 2,6-Di-tert.-butylkresol
0,99 g hypophosphorige Säure (50ï gew.%ig)
0,99 g Triphenylphosphit
0,033 g Phenothiazin

| | |
|---|---|
| abdestillierte Wassermenge: | 111ml |
| Säurezahl: | 37,9mg KOH/g |

### 2. Stufe

106,9 g Diglycidylether von Bisphenol A (Epoxid-Äquivalentgewicht 186g/mol)
18,8 g Tributylamin

| | |
|---|---|
| Produkt-Viskosität (23°C): | 2,6 Pa.s |

### Beispiel 4

Unter Anwendung der in Beispiel 1 beschriebenen zweistufigen Arbeitsweise wurden folgende Verbindungen umgesetzt:

### 1. Stufe:

623,3 g propoxiliertes und ethoxiliertes Trimethylolpropan (PO:EO=86:14;OH-Zahl = 540mg KOH/g)
98,0 g Maleinsäure
316,8 g Acrylsäure
525,1 g Cyclohexan
5,20 g Schwefelsäure
1,04 g Hydrochinonmonomethylether
1,04 g 2,6-Di-tert.-butylkresol
1,04 g hypophosphorige Säure
1,04 g Zinndichloridhydrat
0,52 g Phenothiazin

| | |
|---|---|
| abdestillierte Wassermenge: | 85 ml |
| Säurezahl: | 48,5 mg KOH/g |

### 2. Stufe

128,1 g Pentaerythrittriglycidylether (Epoxid-Äquivalentgewicht 163g/mol)
18,8 g Tributylamin

| | |
|---|---|
| Produkt-Viskosität (23°C ): | 5,36 Pa.s |

Bei den Produkten aus den Beispielen 1 bis 4 konnten gaschromatographisch keine flüchtigen Diolacrylate nachgewiesen werden. Vom Trimethylolpropan abgeleitete Acrylate wurden unter 0.8 Gew.% Anteil ermittelt.

### Vergleichsbeispiel 1:

780g Adipinsäure, 420g Phthalsäureanhydrid, 600g Ethylenglykol und 560g Trimethylolpropan wurden zusammengegeben und auf 160°C aufgeheizt. Anschließend wurde die Temperatur auf 210°C gesteigert und die Veresterung unter Anlegen von Vakuum solange fortgeführt, bis eine Säurezahl von 0,6mg KOH/g erreicht war. Das Produkt hatte eine OH-Zahl von 320mg KOH/g. 1250g davon wurden mit 582g Acrylsäure, 916g Cyclohexan, 5,5g Schwefelsäure, 1,8g Methylhydrochinonmonomethylether, 0,9g 2,6-Di-tert.-butylkresol und 0,04g Phenothiazin zugesetzt. Anschließend wurde weiter Wasser abdestilliert (138g Wasser in 10 Stunden). Nach destillativer Entfernung des Lösungsmittels wies das Reaktionsgemisch eine Säurezahl von 44mg KOH/g auf. Es wurden nun 10,5g Dimethylethanolamin, 192g Pentaerythrittriglycidylether und 1g Thiodiglykol zugegeben und die Reaktion bei 105 bis 110°C fortgeführt. Nach 5 Stunden war eine Säurezahl von 2,6mg KOH/g erreicht. Die Viskosität (23°C ) betrug 47,5 Pa.s.

Nach gaschromatographischen Messungen wurden folgende Mengen an Acrylestern des Glykols und Trimethylolpropans nachgewiesen:
1,0% Hydroxyethylacrylat
3,8% Ethylenglykoldiacrylat
1,9% Trimethylolpropanacrylate

### Prüfung der Lackeigenschaften

Die gemäß den Beispielen hergestellten Produkte wurden nach Verdünnen auf Verarbeitungsviskosität und Zusatz eines Photoinitiators bzw. einer Photoinitiatorkombination in einer 100»m-Schicht (= Nassfilmstärke) auf Glas aufgetragen und in einem Abstand von ca. 10 cm an einer Quecksilbermitteldrucklampe mit einer Leistung von 80 W/cm vorbeigeführt. Die Bestrahlung erfolgte in der Luft. Der in Tabelle 1 für die Reaktivität angegebene Zahlenwert gibt diejenige Bandgeschwindigkeit an, bei der ein kratzfester Überzug erzielt wurde.

**Tabelle 1**

| Lackprüfungen | | | | | |
|---|---|---|---|---|---|
| Polyesteracrylat hergestellt nach Beispiel (je 100g) | 1 | 2 | 3 | 4 | Vergleichsbeispiel 1 |
| Hexandioldiacrylat (g)¹⁾ | 25,3 | 41,6 | 21,4 | 31,6 | 53,8 |
| Hexandioldiacrylat-Anteil (%)¹⁾ | 20,2 | 29,4 | 17,6 | 24,0 | 35,0 |
| Benzildimethylketal | 1,25 | 1,42 | 1,21 | 1,32 | 1,54 |
| Benzophenon | 2,50 | 2,84 | 2,42 | 2,64 | 3,08 |
| Methyldiethanolamin | 3,75 | 4,26 | 3,63 | 3,96 | 4,62 |
| flüchtige Anteile (%) | 3,4 | 3,2 | 3,0 | 2,9 | 7,1 |
| Reaktivität (m/min) | 35 | 30 | 50 | 25 | 45 |
| Pendelhärte (DIN 53 157) | 76 | 97 | 39 | 53 | 42 |

| | | | | | |
|---|---|---|---|---|---|
| ¹⁾ entsprechend einer eingestellten Viskosität von 100 sec Auslaufzeit nach DIN 4 bei 23°C | | | | | |

## Patentansprüche

1. Strahlungshärtbare Acrylate, erhältlich durch glechzeitige Umsetzung von
A) 1 Äquivalent eines 2-bis 6-wertigen oxalkylierten C₂- bis C₁₀-Alkohols mit
B) 0,05 bis 1 Äquivalent einer 2- bis 4-wertigen C₃- bis C₃₆-Carbonsäure oder deren Anhydride und
C) 0,1 bis 1,5 Äquivalenten Acrylsäure und/oder Methacrylsäure
sowie Umsetzung der überschüssigen Carboxylgruppen mit der äquivalenten Menge einer Epoxidverbindung.

2. Strahlungshärtbare Acrylate nach Anspruch 1, erhältlich unter Verwendung von 3- bis 6-wertigen oxalkylierten C₃- bis C₆-Alkoholen als Komponente (A).

3. Strahlungshärtbare Acrylate nach den Ansprüchen 1 oder 2, erhältlich unter Verwendung von oxalkylierten Alkoholen mit einem Oxalkylierungsgrad von 1 bis 30 als Komponente (A).

4. Strahlungshärtbare Acrylate nach einem der Ansprüche 1 bis 3, bei denen die Komponenten (A), (B) und (C) einen Umsetzungsgrad von mindestens 85%, aufweisen.

5. Verfahren zur Herstellung von strahlungshärtbaren Acrylaten gemäß den Ansprüchen 1 bis 4, dadurch gekennzeichnet, daß man
A) 1 Äquivalent eines 2-bis 6-wertigen oxalkylierten C₂- bis C₁₀-Alkohols mit
B) 0,05 bis 1 Äquivalent einer 2- bis 4-wertigen C₃- bis C₃₆-Carbonsäure oder deren Anhydride und
C) 0,1 bis 1,5 Äquivalenten Acrylsäure und/oder Methacrylsäure gleichzeitig
in Gegenwart eines sauren Veresterungskatalysators und mindestens eines Kohlenwasserstoffs, der mit Wasser ein azeotropes Gemisch bildet sowie geringer Mengen eines Polymerisationsinhibitors unter azeotroper Entfernung des entstehenden Wassers bei Temperaturen von 60 bis 140°C verestert, den Kohlenwasserstoff destillativ entfernt und nach Neutralisation des Veresterungskatalysators die überschüssigen Carboxylgruppen mit der äquivalenten Menge einer Epoxidverbindung umsetzt.

6. Verwendung der strahlungshärtbaren Acrylate gemäß einem der Ansprüche 1 bis 4 in strahlungshärtbaren Überzugsmassen.

## Claims

1. A radiation-curable acrylate, obtainable by reacting
A) 1 equivalent of a dihydric to hexahydric oxyalkylated C₂-C₁₀-alcohol simultaneously with
B) from 0.05 to 1 equivalent of a dibasic to tetrabasic C₃-C₃₆-carboxylic acid or its anhydride and
C) from 0.1 to 1.5 equivalents of acrylic acid and/or methacrylic acid
and reacting the excess carboxyl groups with an equivalent amount of an epoxide compound.

2. A radiation-curable acrylate as claimed in claim 1, obtainable using a trihydric to hexahydric oxyalkylated C₃-C₆-alcohol as component (A).

3. A radiation-curable acrylate as claimed in claim 1 or 2, obtainable using an oxyalkylated alcohol having a degree of oxyalkylation of 1 to 30 as component (A).

4. A radiation-curable acrylate as claimed in any of claims 1 to 3, wherein the components (A), (B) and (C) have a conversion of not less then 85%.

5. A process for the preparation of a radiation-curable acrylate as claimed in any of claims 1 to 4, wherein
A) 1 equivalent of a dihydric to hexahydric oxyalkylated C₂-C₁₀-alcohol is esterified simultaneously with
B) from 0.05 to 1 equivalent of a dibasic to tetrabasic C₃-C₃₆-carboxylic acid or its anhydride and
C) from 0.1 to 1.5 equivalents of acrylic acid and/or methacrylic acid
in the presence of an acidic esterification catalyst and one or more hydrocarbons which form an azeotropic mixture with water, as well as a small amount of a polymerization inhibitor, with azeotropic removal of the resulting water, at from 60 to 140°C, the hydrocarbon is removed by distillation and, after neutralization of the esterification catalyst, the excess carboxyl groups are reacted with an equivalent amount of an epoxide compound.

6. Use of a radiation-curable acrylate as claimed in any of claims 1 to 4 in radiation-curable coating materials.

## Revendications

1. Acrylates durcissables par rayonnement, pouvant être obtenus par réaction simultanée de
A) 1 équivalent d'un composé en C₂ à C₁₀ oxalkylé possédant 2 à 6 fois la fonction alcool, avec
B) 0,05 a 1 équivalent d'un acide carboxylique en C₃ à C₃₆ possédant 2 à 4 fois la fonction acide ou de son anhydride et
C) 0,1 à 1,5 équivalent d'acide acrylique et/ou d'acide methacrylique,
ainsi que par reaction des groupements carboxyle en excès avec la quantite équivalente d'un compose époxy.

2. Acrylates durcissables par rayonnement selon la revendication 1, pouvant être obtenus en utilisant, comme composant (A), des composés en C₃ à C₆ oxalkylés possédant 3 à 6 fois la fonction alcool.

3. Acrylates durcissables par rayonnement selon la revendication 1 ou 2, pouvant être obtenus en utilisant, comme composant (A), des alcools oxalkylés ayant un degré d'oxalkylation de 1 à 30.

4. Acrylates durcissables par rayonnement selon l'une quelconque des revendications 1 a 3, dans lesquels les composants (A), (B) et (C) présentent un degré de transformation d'au moins 85%.

5. Procédé de préparation d'acrylates durcissables par rayonnement selon l'une quelconque des revendications 1 à 4, caractérisé en ce qu'on estérifie simultanément
A) 1 équivalent d'un composé en C₂ à C₁₀ oxalkylé possédant 2 à 6 fois la fonction alcool, avec
B) 0,05 à 1 équivalent d'un acide carboxylique en C₃ à C₃₆ possédant 2 à 4 fois la fonction acide ou de son anhydride et
C) 0,1 à 1,5 équivalent d'acide acrylique et/ou d'acide méthacrylique,
en présence d'un catalyseur d'estérification acide et d'au moins un hydrocarbure qui forme un mélange azéotrope avec l'eau, ainsi que de faibles quantités d'un inhibiteur de polymérisation, avec elimination azéotrope de l'eau formee, à des températures de 60 à 140°C, on élimine par distillation l'hydrocarbure et, après neutralisation du catalyseur d'estérification, on fait réagir les groupements carboxyle en excès avec la quantité équivalente d'un composé époxy.

6. Utilisation des acrylates durcissables par rayonnement selon l'une quelconque des revendications 1 à 4 dans des masses à enduire durcissables par rayonnement.
